# FASCICULE DE BREVET EUROPEEN

(11) **EP 2 456 740 B1**
(45) Date de publication et mention de la délivrance du brevet: **24.08.2016**
(21) Numéro de dépôt: 10734501.9
(22) Date de dépôt: 19.05.2010
(51) Int. Cl.: C07C 17/25, C07C 21/18, C07C 11/22

(54) **PROCEDE DE PREPARATION DE COMPOSES FLUORES OLEFINIQUES**
VERFAHREN ZUR HERSTELLUNG VON OLEFINFLUORVERBINDUNGEN
METHOD FOR PREPARING OLEFIN FLUORINE COMPOUNDS

(30) Priorité: 23.07.2009 FR 0955137
(43) Date de publication de la demande: 30.05.2012
(73) Titulaire: Arkema France, 92700 Colombes (FR)
(72) Inventeur: BOSSOUTROT, Jean-Michel, F-69630 Chaponost (FR); SEDAT, Pierre-Marie, F-69210 Fleurieux Sur L'Arbresle (FR)
(74) Mandataire: Dang, Doris
(86) Numéro de dépôt international: PCT/FR2010/050970
(87) Numéro de publication internationale: WO 2011/010023

(56) Documents cités:
- WO-A1-2009/003157
- WO-A2-2008/030439
- WO-A2-2008/030440
- WO-A2-2008/075017
- SIANESI DARIO ET AL: "Fluoro olefins. I. cis- and trans-1,2,3,3,3-Pentafluoropropylene" ANNALI DI CHIMICA, SOCIETA CHIMICA ITALIANA, ROME, IT, vol. 55, no. 8-9, 1 janvier 1965 (1965-01-01), pages 850-861, XP009092725 ISSN: 0003-4592
- DATABASE WPI Week 198035 Thomson Scientific, London, GB; AN 1980-61936C XP002568903 & SU 709 537 A1 (LOPATKINA G A) 25 janvier 1980 (1980-01-25)
- DATABASE WPI Week 200429 Thomson Scientific, London, GB; AN 2004-308185 XP002568913 & JP 2004 018308 A (HASHIMOTO KASEI KK) 22 janvier 2004 (2004-01-22)

## Description

### DOMAINE DE L'INVENTION

L'invention a pour objet un procédé de préparation de composés fluorés oléfiniques. Elle concerne plus particulièrement un procédé de préparation des hydrofluoropropènes.

### ARRIERE-PLAN TECHNOLOGIQUE

Les hydrofluorocarbones (HFC) et en particulier les hydrofluorooléfines (HFOs), telles que le 2,3,3,3-tétrafluoro-1-propène (HFO-1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. A la différence des CFC et des HCFC, qui sont potentiellement dangereux pour la couche d'ozone, les HFOs ne contiennent pas de chlore et donc ne posent pas de problème pour la couche d'ozone.

Le 1,2,3,3,3- pentafluoropropène (HFO-1225ye) est un intermédiaire de synthèse dans la fabrication du 2,3,3,3-tétrafluoro-1-propène (HFO-1234yf).

La plupart des procédés de fabrication des hydrofluorooléfines font appel à une réaction de déhydrohalogénation. Ainsi, le document WO 03/027051 décrit un procédé de fabrication de fluorooléfines de formule CF₃CY=CXₙHₚ, dans laquelle X et Y représentent chacun un atome d'hydrogène ou d'halogène choisi parmi le fluor, le chlore, le brome ou l'iode ; n et p sont des nombres entiers et peuvent indépendamment prendre la valeur zéro, 1 ou 2 à condition que (n + p) = 2, comprenant la mise en contact d'un composé de formule CF₃C(R¹ₐR²_{b})C(R³_{c}R⁴_{d}) avec R¹, R², R³ et R⁴ représentant indépendamment un atome d'hydrogène ou d'halogène choisi parmi le fluor, le chlore, le brome ou l'iode à condition qu'au moins un de R¹, R², R³ et R⁴ est un atome d'halogène et qu'au moins un atome d'hydrogène et un atome d'halogène sont situés sur des atomes de carbone adjacent ; a et b peuvent indépendamment prendre la valeur zéro, 1 ou 2 à condition que (a + b) = 2 ; c et d peuvent indépendamment prendre la valeur zéro, 1, 2 ou 3 à condition que (c + d) = 3, avec au moins un hydroxyde de métal alcalin en présence d'un catalyseur de transfert phase.

Ce document enseigne à l'exemple 2, qu'en l'absence d'un catalyseur de transfert de phase, il n'y a pas de réaction lorsque le 1,1,1,3,3-pentafluoropropane (HFC-245fa) est mis en contact avec une solution aqueuse de 50 % en poids de hydroxyde de potassium (KOH) pendant 24 heures à température ambiante et sous pression.

Ce document enseigne en outre une température de réaction compris entre-20°C et 80°C.

Le document WO 2008/075017 illustre la réaction de déhydrofluoration du 1,1,1,2,3,3-hexafluoropropane (HFC-236ea) en 1,2,3,3,3-pentafluoropropène (HFO-1225ye) à 150° C en présence d'une solution aqueuse de 50 % en poids de KOH. En l'absence d'un catalyseur de transfert de phase, la conversion au bout de 3 heures et demie est de 57,8 % et la sélectivité en HFO-1225ye est de 52,4 % (essai 1). En présence d'un catalyseur de transfert de phase, cette conversion est atteinte au bout de 2,5 heures seulement et la sélectivité est pratiquement inchangée (essai 4). Comme indiqué au tableau 2 de ce document pour accroître la sélectivité en HFO-1225ye, il est nécessaire d'utiliser un solvant organique.

WO 2007/056194 décrit la préparation de HFO-1234yf par déhydrofluoration du 1,1,1,2,3-pentafluoropropane (HFC-245eb) soit avec une solution aqueuse de KOH soit en phase gazeuse en présence d'un catalyseur, notamment sur catalyseur à base de nickel, de carbone ou une combinaison de ceux-ci.

Le document Knunyants et al, Journal of the USSR Academy of Sciences, Chemistry Department, "reactions of fluoro-olefins", report 13., "catalytic hydrogenation of perfluoro-olefins", 1960, décrit de façon distincte diverses réactions chimiques sur des composes fluorés. Ce document décrit la déhydrofluoration du 1,1,1,2,3,3-hexafluoropropane (236ea) par passage au travers d'une suspension de KOH en poudre dans l'éther de dibutyle, pour produire du 1,2,3,3,3-pentafluoropropène-1 (HFO-1225ye) avec un rendement de 60 % seulement. Ce document décrit également la déhydrofluoration de 1,1,1,2,3-pentafluoropropane (HFC-245eb) en 2,3,3,3-tétrafluoro-1-propène (HFO-1234yf) par passage dans une suspension de KOH en poudre dans l'éther de dibutyle avec un rendement de 70 % seulement.

Par ailleurs, la figure 2 à la page 51 du deuxième fascicule du nouveau traité de chimie minérale de P. Pascal, Ed. 1963, montre l'allure des équilibres liquide solide du système eau et hydroxyde de potassium et les mesures sont rassemblées dans le tableau à la page 52.

Les réactions de déhydrofluoration telles que décrites ci-dessus conduisent, outre au composé hydrofluorooléfinique recherché, à la formation de l'eau et de fluorure de potassium. Par ailleurs, la mise en oeuvre d'une telle réaction en continu n'est pas aisée à l'échelle industrielle car au moins trois phases (gazeuse, liquide et solide) sont mises en jeu.

Les documents SU 709 537 et JP 2004 018308 divulguent la production de CaF₂ par traitement d'une solution de KF en présence de Ca(OH)₂.

La présente invention fournit un procédé de fabrication continu ou semi-continu d'un composé (hydro)fluorooléfinique permettant de remédier aux inconvénients précités. La présente invention a donc pour objet un procédé de fabrication continu ou semi-continu d'un composé (hydro)fluorooléfinique comprenant (i) la mise en contact dans un réacteur agité, muni d'au moins une entrée pour les réactifs et d'au moins une sortie, d'au moins un composé comprenant de trois à six atomes de carbone, d'au moins deux atomes de fluor et d'au moins un atome d'hydrogène, à la condition qu' au moins un atome d'hydrogène et un atome de fluor sont situés sur des atomes de carbone adjacent, avec de l'hydroxyde de potassium dans un milieu réactionnel aqueux, pour donner le composé (hydro)fluorooléfinique, qui est séparé du milieu réactionnel sous forme gazeux, et du fluorure de potassium, (ii) la mise en contact en milieu aqueux du fluorure de potassium formé en (i) avec de l'hydroxyde de calcium dans un deuxième réacteur pour donner de l'hydroxyde de potassium et précipiter du fluorure de calcium, (iii) séparation du fluorure de calcium précipité à l'étape (ii) du milieu réactionnel et (iv) éventuellement le milieu réactionnel est recyclé après éventuel ajustement de la concentration en hydroxyde de potassium à l'étape (i) caractérisé en ce que l'hydroxyde de potassium représente dans le milieu réactionnel de l'étape (ii) entre 10 et 35 % en poids par rapport au poids du mélange eau et hydroxyde de potassium du milieu.

La présente invention permet ainsi d'obtenir un procédé avantageux car d'une part, l'hydroxyde de potassium est plus réactive que l'hydroxyde de calcium dans la réaction de déhydrofluoration et d'autre part, la conversion de l'hydroxyde de calcium en fluorure de calcium, sous produit valorisable est élevée.

La demanderesse a observé que le procédé selon la présente invention permet d'obtenir une taille moyenne à 50 % en poids de la distribution granulométrique de cristaux de fluorure de calcium supérieure à 10 µm, voire supérieure à 20 µm et plus particulièrement comprise entre 20 et 60 µm et ainsi faciliter les opérations de lavage, filtration et le recyclage de l'hydroxyde de potassium.

Le milieu réactionnel de l'étape (i) est agité de manière à assurer une dispersion du gaz dans le milieu liquide.

Le procédé selon la présente invention fournit de préférence un composé (hydro)fluorooléfinique comportant trois atomes de carbone, avantageusement un composé (hydro)fluorooléfinique représenté par la formule (I)

CF₃CY=CXₙHₚ (I)

dans laquelle Y représente un atome d'hydrogène ou d'halogène choisi parmi le fluor, le chlore, le brome ou l'iode et X représente un atome d'halogène choisi parmi le fluor, le chlore, le brome ou l'iode ; n et p sont des nombres entiers et peuvent indépendamment prendre la valeur zéro, 1 ou 2 à condition que (n + p) = 2, en mettant en contact un composé de formule CF₃CYRCR'XₙHₚ, dans laquelle X, Y, n et p ont la même signification que dans la formule (I) et R représente un atome de fluor lorsque R' représente un atome d'hydrogène ou R représente un atome d'hydrogène lorsque R' représente un atome de fluor, avec l'hydroxyde de potassium dans l'étape (i).

La présente invention convient tout particulièrement à la fabrication d'un composé de formule (Ia)

CF₃-CF=CHZ (Ia)

dans laquelle Z représente un atome d'hydrogène ou fluor à partir d'un composé de formule CF₃CFRCHR'Z dans laquelle Z a la même signification que dans la formule (Ia) et R représente un atome de fluor lorsque R' représente un atome d'hydrogène ou R représente un atome d'hydrogène lorsque R' représente un atome de fluor.

Ainsi, le 2,3,3,3-tetrafluoropropène peut être obtenu par déhydrofluoration du 1,2,3,3,3-pentafluoropropane avec le KOH et/ou 1,2,3,3,3-pentafluoropropène par déhydrofluoration du 1,1,1,2,3,3-hexafluoropropane avec le KOH dans l'étape (i). Le 1,2,3,3,3-pentafluoropropène peut être sous la forme d'isomère cis et/ou trans.

La présente invention peut en outre être utilisée pour la fabrication du 1,3,3,3-tetrafluoropropène par déhydrofluoration du 1,1,3,3,3-pentafluoropropane avec le KOH.

Dans l'étape (i) du procédé selon la présente invention l'hydroxyde de potassium peut représenter entre 20 et 75 % en poids par rapport au poids du mélange eau et KOH présent dans le milieu réactionnel aqueux, de préférence comprise entre 55 et 70 %. Suivant la teneur, l'hydroxyde de potassium peut être sous forme de solution aqueuse ou à l'état fondu.

L'étape (i) est en général mise en oeuvre à une température telle que l'eau formée en cours de réaction de déhydrofluoration est éliminée, en partie ou en totalité, du milieu réactionnel par entraînement du flux gazeux comprenant le composé (hydro)fluorooléfinique, issu du réacteur agité. Cette température est de préférence comprise entre 80 et 180° C, avantageusement comprise entre 125 et 180° C, et tout particulièrement comprise entre 145 et 165° C.

La réaction de déhydrofluoration de l'étape (i) peut être mise en oeuvre à pression atmosphérique mais on préfère travailler à une pression supérieure à la pression atmosphérique. Avantageusement, cette pression est comprise entre 1,1 et 2,5 bars.

La réaction de l'étape (ii) peut être mise en oeuvre dans un réacteur agité ou à lit fluidisé en faisant réagir de l'hydroxyde de calcium, de préférence en suspension dans l'eau, avec le fluorure de potassium en provenance de l'étape (i). La température de réaction peut varier dans des larges limites mais pour des raisons économiques, elle est de préférence comprise entre 50 et 150 ° C, avantageusement entre 70 et 120° C et plus avantageusement entre 70 et 100 ° C.

Lorsqu'une suspension d'hydroxyde de calcium est utilisée à l'étape (ii), l'hydroxyde de calcium représente entre 2 et 40 % en poids par rapport au poids de la suspension.

Avantageusement, l'étape (ii) est alimentée en fluorure de potassium par le milieu réactionnel provenant de l'étape (i) comprenant de l'eau, de l'hydroxyde de potassium et du fluorure de potassium. Le fluorure de potassium dans l'étape (i) peut être dissous ou en suspension. Le fluorure de potassium représente de préférence entre 4 et 45 % en poids du milieu réactionnel de l'étape (i).

Dans l'étape (ii) deux moles de fluorure de potassium réagissent avec un mole d'hydroxyde de calcium pour donner une mole de fluorure de potassium et deux moles d'hydroxyde de potassium. Cette génération d'hydroxyde de potassium permet de limiter le besoin éventuel de re-concentration et diminue ainsi l'ajout d'hydroxyde de potassium dans le procédé.

On peut prévoir une étape de dilution du milieu réactionnel entre l'étape (i) et l'étape (ii).

Le fluorure de calcium précipité à l'étape (ii) est séparé du milieu réactionnel, par exemple par filtration et/ou décantation. Préalablement à la filtration, on peut prévoir une étape de décantation. Le fluorure de calcium ainsi séparé est ensuite lavé à l'eau.

Au cours de l'étape de décantation, on peut prévoir le recyclage d'une partie de la suspension concentrée en fluorure de calcium à l'étape (ii). Avantageusement, le taux de solides de fluorure de calcium présent dans le milieu réactionnel de l'étape (ii) est compris entre 2 et 30 % en poids.

Après séparation du fluorure de calcium, le milieu réactionnel avec ou sans eaux de lavage du fluorure de calcium peut être recyclé à l'étape (i) après éventuel ajustement de la teneur en hydroxyde de potassium.

Il peut être avantageux d'utiliser un gaz inerte dans l'étape de déhydrofluoration.

Le procédé selon la présente invention a l'avantage de conduire à des rendements élevés même en l'absence de catalyseur de transfert de phase et/ou de solvant organique.

La présente invention comprend également les combinaisons des formes préférées quel que soit le mode de réalisation.

### PARTIE EXPERIMENTALE

### Exemple 1

Dans un réacteur, on introduit 1 Kg de potasse à 50 % en poids contenant 9% en poids de KF et on chauffe à 100° C. On ajoute ensuite sous agitation de 500 tours/min, 109g de Ca(OH)₂ titrant 96 % en poids (impureté majeure étant CaCO₃). Au bout d'une heure de réaction, on prélève la suspension. Le taux de solide est de 3,5 % en poids et la composition du solide en poids est la suivante :
CaF₂ : 60 %
Ca(OH)₂ : 36%
CaCO₃ : 4%

### Exemple 2

On opère comme à l'exemple 1 sauf que l'on introduit 1 Kg de potasse à 25 % en poids.
la composition du solide en poids, au bout d'une heure de réaction, est la suivante :
CaFz : 95 %
Ca(OH)₂ : 1%
CaCO₃ : 4%.

### Exemple 3

On alimente en continu un réacteur maintenu à 100° C et agité à 500 tr/min par une solution de potasse issue de l'étape de déhydrofluoration et titrant après dilution 28% en poids en potasse et 6% en poids KF. La suspension de Ca(OH)₂ alimentant le réacteur titre 20 % poids. Le temps de séjour dans le réacteur est d'environ 1h.
La filtrabilité de la suspension obtenue après réaction est très bonne.
Le taux de solide de la suspension en sortie du réacteur est de 3,6% en poids.
La granulométrie du fluorure de calcium synthétisé est de 30 µm et sa pureté supérieure à 85% poids.

### Exemple 4

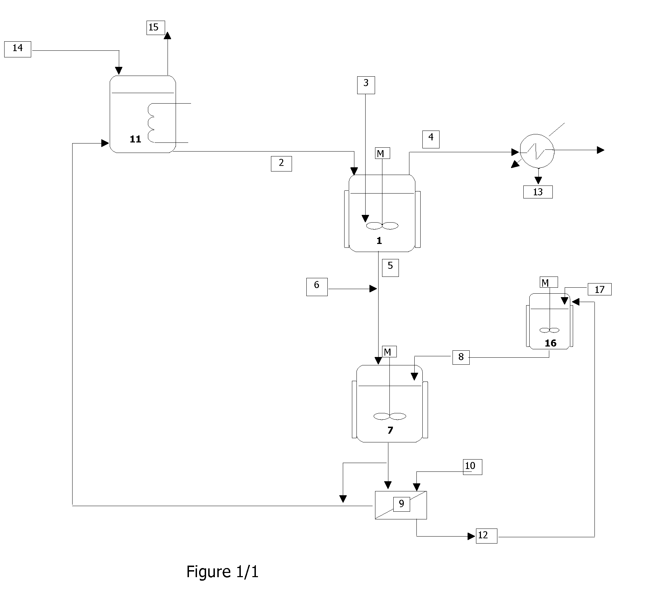

La figure 1 donne le schéma d'un mode de réalisation de la présente invention. Un réacteur agité (1), équipé d'un dispositif de chauffage/refroidissement et de mesure de température du milieu réactionnel, contenant un mélange eau et KOH dans lequel le KOH est présent à 60 % en poids dans l'eau, est alimenté en continu par une solution de KOH fondu (2) dans lequel le KOH est présent à 65 % en poids dans l'eau, et par du 1,1,1,2,3,3-hexafluoropropane (3). La température est maintenue à 150° C et la pression dans le réacteur est de 1,2 bars absolus. Les produits gazeux sortent du réacteur par un orifice (4) situé sur le couvercle et l'eau contenue dans le flux gazeux est éliminé par condensation (13).

La sortie (5) du réacteur (1) est diluée en ligne par de l'eau (6) pour obtenir un titre en KOH de 30%. Ce mélange est envoyé à l'entrée du réacteur (7) et assure donc l'alimentation du réacteur (7) en fluorure de potassium, pouvant être en suspension dans le milieu aqueux. Une suspension de 15 % en poids d'hydroxyde de calcium dans l'eau est introduite dans le réacteur (7) par la voie (8). Le réacteur (7) est maintenu à une température comprise entre 70 et 80 ° C.

La sortie du réacteur (7) est reliée à un filtre (9) pour séparer le fluorure de calcium du milieu réactionnel, puis le laver à l'eau (10) ; le milieu aqueux séparé du fluorure de calcium est ensuite recyclé au réacteur (1) après ajustement de la concentration du KOH. Les eaux de lavage du florure de calcium sont recyclées au bac (16) de préparation de la suspension d'hydroxyde de calcium dans l'eau.

Le mélange de KOH fondu alimentant le réacteur (1) est préparé par évaporation ( élimination d'eau (15)) d'une solution aqueuse de 50 % en poids de KOH (14) et de la solution aqueuse provenant du filtre (9).

En sortie du réacteur (1), le taux de conversion molaire du 1,1,1,2,3,3-hexafluoropropane est supérieure à 98 %. La sélectivité en 1,1,1,2,3 pentafluoropropène est supérieure à 99 %.

En sortie du réacteur (7), le taux de conversion molaire de l'hydroxyde de calcium est supérieure à 85 %.

### Exemple 5

On opère comme à l'exemple 4 sauf que l'on alimente le réacteur (1) en continu par du 1,2,3,3,3-pentafluoropropane à la place du 1,1,1,2,3,3-hexafluoropropane.

Le réacteur agité (1) contient un mélange eau et KOH dans lequel le KOH est présent à 65 % en poids dans l'eau.

En sortie du réacteur (1), le taux de conversion molaire du 1,2,3,3,3-pentafluoropropane est supérieure à 98 %. La sélectivité en 1,1,1,2 tetrafluoropropène est supérieure à 99 %.

### Exemple 6

On alimente en continu un réacteur maintenu à 80° C et agité à 500 tr/min par une solution de potasse issue de l'étape de déhydrofluoration et titrant après dilution 32,8% en poids en potasse et 9,7% en poids KF. La suspension de Ca(OH)₂ alimentant le réacteur titre 15% poids. Le temps de séjour dans le réacteur est d'environ 1h.

La filtrabilité de la suspension obtenue après réaction est très bonne.

Le taux de solide de la suspension en sortie du réacteur est de 3,6% en poids.

La granulométrie du fluorure de calcium synthétisé est de 30 µm et sa pureté supérieure à 85% poids.

## Revendications

1. Procédé de fabrication continu ou semi-continu d'un composé (hydro)fluorooléfinique comprenant (i) la mise en contact dans un réacteur agité, muni d'au moins une entrée pour les réactifs et d'au moins une sortie, d'au moins un composé comprenant de trois à six atomes de carbone, d'au moins deux atomes de fluor et d'au moins un atome d'hydrogène, à la condition qu' au moins un atome d'hydrogène et un atome de fluor sont situés sur des atomes de carbone adjacent, avec de l'hydroxyde de potassium dans un milieu réactionnel aqueux, pour donner le composé (hydro)fluorooléfinique, qui est séparé du milieu réactionnel sous forme gazeux, et du fluorure de potassium, (ii) la mise en contact en milieu aqueux du fluorure de potassium formé en (i) avec de l'hydroxyde de calcium dans un deuxième réacteur pour donner de l'hydroxyde de potassium et précipiter du fluorure de calcium, (iii) séparation du fluorure de calcium précipité à l'étape (ii) du milieu réactionnel et (iv) éventuellement le milieu réactionnel est recyclé après éventuel ajustement de la concentration en hydroxyde de potassium à l'étape (i) **caractérisé en ce que** l'hydroxyde de potassium représente dans le milieu réactionnel de l'étape (ii) entre 10 et 35 % en poids par rapport au poids du mélange eau et hydroxyde de potassium du milieu.

2. Procédé selon la revendication 1 **caractérisé en ce que** le composé (hydro)fluorooléfinique de formule (I)
CF₃CY=CXₙHₚ (I)
dans laquelle Y représente un atome d'hydrogène ou d'halogène choisi parmi le fluor, le chlore, le brome ou l'iode et X représente un atome d'halogène choisi parmi le fluor, le chlore, le brome ou l'iode ; n et p sont des nombres entiers et peuvent indépendamment prendre la valeur zéro, 1 ou 2 à condition que (n + p) = 2, est obtenu par la mise en contact d' un composé de formule CF₃CYRCR'XₙHₚ, dans laquelle X, Y, n et p ont la même signification que dans la formule (I) et R représente un atome de fluor lorsque R' représente un atome d'hydrogène ou R représente un atome d'hydrogène lorsque R' représente un atome de fluor, avec l'hydroxyde de potassium dans l'étape (i).

3. Procédé selon la revendication 1 **caractérisé en ce que** le composé (hydro)fluorooléfinique est de formule (Ia)
CF₃-CF=CHZ (Ia)
dans laquelle Z représente un atome d'hydrogène ou fluor comprend la mise en contact d'un composé de formule CF₃CFRCHR'Z, dans laquelle Z a la même signification que dans la formule (Ia) et R représente un atome de fluor lorsque R' représente un atome d'hydrogène ou R représente un atome d'hydrogène lorsque R' représente un atome de fluor, avec l'hydroxyde de potassium dans l'étape (i).

4. Procédé selon l'une quelconque des revendications précédentes **caractérisé en ce que** le 2,3,3,3-tetrafluoropropène est obtenu par la mise en contact du 1,2,3,3,3-pentafluoropropane avec de l'hydroxyde de potassium et/ou le 1,2,3,3,3-pentafluoropropène par la mise en contact du 1,1,1,2,3,3-hexafluoropropane avec de l'hydroxyde de potassium dans l'étape (i).

5. Procédé selon l'une quelconque des revendications précédentes **caractérisé en ce que** l'hydroxyde de potassium peut représenter entre 20 et 75 % en poids par rapport au poids du mélange eau et KOH présent dans le milieu réactionnel aqueux de l'étape (i), de préférence comprise entre 55 et 70 % en poids.

6. Procédé selon l'une quelconque des revendications précédentes **caractérisé en ce que** la température de mise en oeuvre de l'étape (i) est comprise entre 80 et 180° C, de préférence comprise entre 125 et 180° C, et avantageusement comprise entre 145 et 165° C.

7. Procédé selon l'une quelconque des revendications précédentes **caractérisé en ce que** la température de l'étape (ii) est comprise entre 50 et 150 ° C et de préférence entre 70 et 120° C et avantageusement entre 70 et 100° C.

8. Procédé selon l'une quelconque des revendications précédentes **caractérisé en ce que** l'étape (ii) est alimentée en fluorure de potassium par le milieu réactionnel en provenance de l'étape (i).

9. Procédé selon l'une quelconque des revendications précédentes **caractérisé en ce que** le fluorure de potassium représente entre 4 et 45 % en poids du milieu réactionnel de l'étape (i).

10. Procédé selon la revendication 8 **caractérisé en ce que** l'on ajoute de l'eau dans le milieu réactionnel de l'étape (ii).

11. Procédé selon l'une quelconque des revendications précédentes **caractérisé en ce que** le fluorure de calcium à l'étape (iii) est filtré après une éventuelle étape de décantation.

12. Procédé selon la revendication 11 **caractérisé en ce que** lors de décantation une partie de la suspension concentrée en fluorure de calcium est recyclé à l'étape (ii).

## Patentansprüche

1. Verfahren zur kontinuierlichen oder halbkontinuierlichen Herstellung einer (Hydro)fluorolefinverbindung, das Folgendes umfasst: (i) Inberührungbringen mindestens einer Verbindung mit drei bis sechs Kohlenstoffatomen, mindestens zwei Fluoratomen und mindestens einem Wasserstoffatom, mit der Maßgabe, dass sich mindestens ein Wasserstoffatom und ein Fluoratom an benachbarten Kohlenstoffatomen befinden, mit Kaliumhydroxid in einem wässrigen Reaktionsmedium in einem Rührreaktor mit mindestens einem Einlass für die Reaktanten und mindestens einem Auslass zum Erhalt der (Hydro)fluorolefinverbindung, die in gasförmiger Form von dem Reaktionsmedium abgetrennt wird, und von Kaliumfluorid, (ii) Inberührungbringen des in (i) gebildeten Kaliumfluorids mit Calciumhydroxid in wässrigem Medium in einem zweiten Reaktor zum Erhalt von Kaliumhydroxid und zur Ausfällung von Calciumfluorid, (iii) Abtrennen des in Schritt (ii) ausgefällten Calciumfluorids von dem Reaktionsmedium und (iv) gegebenenfalls Rezyklieren des Reaktionsmediums nach fakultativer Einstellung der Kaliumhydroxid-Konzentration in Schritt (i), **dadurch gekennzeichnet, dass** das Kaliumhydroxid in dem Reaktionsmedium von Schritt (ii) zwischen 10 und 35 Gew.-%, bezogen auf das Gewicht der Mischung von Wasser und Kaliumhydroxid des Mediums, ausmacht.

2. Verfahren nach Anspruch 1, **dadurch gekennzeichnet, dass** die (Hydro)fluorolefinverbindung der Formel (I)
CF₃CY=CXₙHₚ (I)
in der Y für ein Wasserstoffatom oder ein aus Fluor, Chlor, Brom oder Iod ausgewähltes Halogenatom steht, X für ein aus Fluor, Chlor, Brom oder Iod ausgewähltes Halogenatom steht und n und p ganze Zahlen sind und unabhängig den Wert null, 1 oder 2 annehmen können, mit der Maßgabe, dass (n + p) = 2, durch Inberührungbringen einer Verbindung der Formel CF₃CYRCR'XₙHₚ, in der X, Y, n und p die gleiche Bedeutung wie in Formel (I) besitzen und R für ein Fluoratom steht, wenn R' für ein Wasserstoffatom steht, oder R für ein Wasserstoffatom steht, wenn R' für ein Fluoratom steht, mit Kaliumhydroxid in Schritt (i) erhalten wird.

3. Verfahren nach Anspruch 1, **dadurch gekennzeichnet, dass** die (Hydro)fluorolefinverbindung die Formel (Ia) aufweist :
CF₃-CF=CHZ (Ia)
in der Z für ein Wasserstoff- oder Fluoratom steht, das Inberührungbringen einer Verbindung der Formel CF₃CFRCHR'Z, in der Z die gleiche Bedeutung wie in Formel (Ia) besitzt und R für ein Fluoratom steht, wenn R' für ein Wasserstoffatom steht, oder R für ein Wasserstoffatom steht, wenn R' für ein Fluoratom steht, mit Kaliumhydroxid in Schritt (i) umfasst.

4. Verfahren nach einem der vorhergehenden Ansprüche, **dadurch gekennzeichnet, dass** in Schritt (i) durch Inberührungbringen von 1,2,3,3,3-Pentafluorpropan mit Kaliumhydroxid 2,3,3,3-Tetrafluorpropen und/oder durch Inberührungbringen von 1,1,1,2,3,3-Hexafluorpropan mit Kaliumhydroxid 1,2,3,3,3-Pentafluorpropen erhalten wird.

5. Verfahren nach einem der vorhergehenden Ansprüche, **dadurch gekennzeichnet, dass** das Kaliumhydroxid zwischen 20 und 75 Gew.-%, bezogen auf das Gewicht der Mischung von Wasser und KOH in dem wässrigen Reaktionsmedium von Schritt (i), und vorzugweise zwischen 55 und 70 Ges.-% ausmachen kann.

6. Verfahren nach einem der vorhergehenden Ansprüche, **dadurch gekennzeichnet, dass** die Temperatur, bei der Schritt (i) durchgeführt wird, zwischen 80 und 180°C, vorzugweise zwischen 125 und 180°C und vorteilhafterweise zwischen 145 und 165°C liegt.

7. Verfahren nach einem der vorhergehenden Ansprüche, **dadurch gekennzeichnet, dass** die Temperatur von Schritt (ii) zwischen 50 und 150°C und vorzugweise zwischen 70 und 120°C und vorteilhafterweise zwischen 70 und 100°C liegt.

8. Verfahren nach einem der vorhergehenden Ansprüche, **dadurch gekennzeichnet, dass** Schritt (ii) durch das Reaktionsmedium aus Schritt (i) mit Kaliumfluorid versorgt wird.

9. Verfahren nach einem der vorhergehenden Ansprüche, **dadurch gekennzeichnet, dass** das Kaliumfluorid zwischen 4 und 45 Gew.-% des Reaktionsmediums von Schritt (i) ausmacht.

10. Verfahren nach Anspruch 8, **dadurch gekennzeichnet, dass** man Wasser zu dem Reaktionsmedium von Schritt (ii) gibt.

11. Verfahren nach einem der vorhergehenden Ansprüche, **dadurch gekennzeichnet, dass** das Calciumfluorid aus Schritt (iii) nach einem fakultativen Absetzschritt filtriert wird.

12. Verfahren nach Anspruch 11, **dadurch gekennzeichnet, dass** während des Absetzens ein Teil der konzentrierten Calciumfluorid-Suspension in den Schritt (ii) rezykliert wird.

## Claims

1. Process for the continuous or semicontinuous manufacture of a (hydro)fluoroolefin compound comprising (i) bringing at least one compound comprising from three to six carbon atoms, at least two fluorine atoms and at least one hydrogen atom, provided that at least one hydrogen atom and one fluorine atom are situated on adjacent carbon atoms, into contact with potassium hydroxide in an aqueous reaction medium in a stirred reactor equipped with at least one inlet for the reactants and with at least one outlet, to give the (hydro)fluoroolefin compound, which is separated from the reaction medium in the gaseous form, and potassium fluoride, (ii) bringing the potassium fluoride formed in (i) into contact in an aqueous medium with calcium hydroxide in a second reactor, to give potassium hydroxide and to precipitate calcium fluoride, (iii) separating the calcium fluoride precipitated in stage (ii) from the reaction medium and (iv) optionally recycling the reaction medium to stage (i) after optional adjustment of the concentration of potassium hydroxide, **characterized in that** the potassium hydroxide represents, in the reaction medium of stage (ii), between 10 and 35% by weight, with respect to the weight of the water and potassium hydroxide mixture of the medium.

2. Process according to Claim 1, **characterized in that** the (hydro)fluoroolefin compound of formula (I)
CF₃CY=CXₙHₚ (I)
in which Y represents a hydrogen atom or a halogen atom chosen from fluorine, chlorine, bromine or iodine, X represents a halogen atom chosen from fluorine, chlorine, bromine or iodine, and n and p are integers and can independently take the value zero, 1 or 2, provided that (n + p) = 2, is obtained by bringing a compound of formula CF₃CYRCR' XₙHₚ, in which X, Y, n and p have the same meanings as in the formula (I) and R represents a fluorine atom when R' represents a hydrogen atom or R represents a hydrogen atom when R' represents a fluorine atom, into contact with potassium hydroxide in stage (i).

3. Process according to Claim 1, **characterized in that** the (hydro)fluoroolefin compound is of formula (Ia)
CF₃-CF=CHZ (Ia)
in which Z represents a hydrogen atom or a fluorine atom, comprises bringing a compound of formula CF₃CFRCHR'Z, in which Z has the same meanings as in the formula (Ia) and R represents a fluorine atom when R' represents a hydrogen atom or R represents a hydrogen atom when R' represents a fluorine atom, into contact with potassium hydroxide in stage (i).

4. Process according to any one of the preceding claims, **characterized in that** 2,3,3,3-tetrafluoropropene is obtained by bringing 1,1,1,2,3-pentafluoropropane into contact with potassium hydroxide and/or 1,2,3,3,3-pentafluoropropene is obtained by bringing 1,1,1,2,3,3-hexafluoropropane into contact with potassium hydroxide in stage (i).

5. Process according to any one of the preceding claims, **characterized in that** the potassium hydroxide can represent between 20 and 75% by weight, with respect to the weight of the water and KOH mixture present in the aqueous reaction medium of stage (i), preferably between 55 and 70% by weight.

6. Process according to any one of the preceding claims, **characterized in that** the temperature at which stage (i) is carried out is between 80 and 180°C, preferably between 125 and 180°C and advantageously between 145 and 165°C.

7. Process according to any one of the preceding claims, **characterized in that** the temperature of stage (ii) is between 50 and 150°C, preferably between 70 and 120°C and advantageously between 70 and 100°C.

8. Process according to any one of the preceding claims, **characterized in that** stage (ii) is fed with potassium fluoride via the reaction medium originating from stage (i).

9. Process according to any one of the preceding claims, **characterized in that** the potassium fluoride represents between 4 and 45% by weight of the reaction medium from stage (i).

10. Process according to Claim 8, **characterized in that** water is added to the reaction medium of stage (ii).

11. Process according to any one of the preceding claims, **characterized in that** the calcium fluoride in stage (iii) is filtered off after an optional settling stage.

12. Process according to Claim 11, **characterized in that**, during settling, a portion of the concentrated calcium fluoride suspension is recycled to stage (ii).
